# EUROPEAN PATENT SPECIFICATION

(11) **EP 2 170 168 B1**
(45) Date of publication and mention of the grant of the patent: **21.12.2011**
(21) Application number: 08773483.6
(22) Date of filing: 17.06.2008
(51) Int. Cl.: A61B 5/11

(54) **TRANSPARENT BED FOR SELF-CORRECTION OF THE SPINAL COLUMN THROUGH A VIDEO CAMERA AND COMPUTER SYSTEM**
TRANSPARENTES BETT ZUR SELBSTKORREKTUR DER WIRBELSÄULE ÜBER EINE VIDEOKAMERA UND EIN COMPUTERSYSTEM
LIT TRANSPARENT POUR AUTO-CORRECTION DE LA COLONNE SPINALE PAR UN SYSTEME DE CAMERA VIDEO ET D'ORDINATEUR

(30) Priority: 26.06.2007 IT LT20070005
(43) Date of publication of application: 07.04.2010
(73) Proprietor: Istituto Di Fisioterapia E Terapia Manuale Di Prosseda, Mariano, 04100 Latina (IT)
(72) Inventor: PROSSEDA, Mariano, I-04010 Norma (IT)
(74) Representative: Statti, Francesco
(86) International application number: PCT/EP2008/004863
(87) International publication number: WO 2009/000444

(56) References cited:
- EP-A- 0 074 231

## Description

### Field of invention

The present invention concerns the medical sector and more specifically the therapeutic sector for the rehabilitation of the vertebral column and it concerns a transparent rehabilitation bed equipped with a system comprising a video camera, a computer and a monitor which enables the patient from a lying supine position to see his/her own spinal column in a dynamic situation so as to be able to take an active part in the rehabilitation treatment also facilitating the work of the therapist who thus has interaction between the diagnostic and therapeutic moment as well as a perfect visual perception of the back during therapeutic manipulation so as to allow optimization of the results obtained from the therapy itself.

### Background

Among the various therapies for diseases of the spinal column such as for example vertebral dysmorphism or paramorphism, scoliosis or other problems, rehabilitation treatment also consists of the realignment of the vertebral arches of the patient by stretching the internal muscles. e.g. through a special type of breathing suggested by the therapist. For this reason, it is essential for the patient to take an active part in the therapy session and to understand, first and foremost, how to perform the correction exercises of his/her own back as suggested by the therapist and under the guidance of the same.

Currently, this need is fulfilled by showing the patient his/her spinal disease through an X-ray, a photograph, a Nuclear Magnetic Resonance image, or a Computerized Axial Tomography but in all cases in a static situation which does not help the patient during the therapy session to understand the significance of the positions suggested by the therapist to rebalance his/her spinal column. As a result, one of the major problems that the therapist faces during rehabilitation sessions is lack of participation on the part of the patient, especially in the case of children and adolescents, as it is impossible for them to have a dynamic vision of how their spinal column actually changes as a result of the corrective positions suggested by the therapist and therefore to cooperate effectively with the latter during the rehabilitation session.

The therapist, moreover, currently encounters difficulties in the execution of the corrective procedure because he/she has a static vision of the patient's spinal column which does not allow him/her interaction between the diagnostic and therapeutic moment.

Additionally, the therapist, when carrying out his/her rehabilitative work on a patient lying on a normal bed, is guided exclusively by the sensations that the patient expresses and, therefore, by the latter's personal powers of description, linked to his/her subjective state and not by an objective examination of the part under treatment.

The background art document EP 0 074 231 A discloses a combination of interrelated means (frame, transparent track, mirror, rails, support, one or more cameras) to facilitate the viewing and recording of dynamic podologic activities in order to carry out orthopedic studies. This recording follows the same direction of the patient's feet and with the same speed, therefore it is dynamic thanks to the movements of the patient, who however does not watch what the camera is taping and whose sole function is that of walking on the transparent track.

In fact the video screens to which the cameras are connected reproduce a series of images which can be watched, stopped and played back by the therapist in order to be studied and analysed, but there cannot be an active patient's involvement in the rehabilitation treatment.

### Disclosure of invention

It is an object of the present invention to resolve the problems presently called to mind by proposing a bed with a transparent base for the patient to lie on, equipped with a video camera and computer system that will give the patient a dynamic vision of his/her back when lying in a supine position during the therapy session.

It is another object of the present invention to improve cooperation between the therapist and the patient who, being able to view his/her back in a lying position and during the dynamic phase, is thus empowered to self-correct the position as desired and suggested by the said therapist.

It is another object of the present invention to motivate patients, especially children affected by vertebral problems, to take a greater interest in the problems of their own back, by capturing their attention as though they were in front of a video game and helping them participate actively in the therapy session.

It is another object of the present invention to simplify the therapist's work and render it more efficient by allowing the latter to view, together with the patient, the spinal column in its lying supine position and during the dynamic phase with a view to performing the best possible corrective procedure, as well as guaranteeing interaction between the diagnostic and therapeutic moment and a perfect visual perception of the back during therapeutic manipulation.

These and other objects are achieved according to the invention that is the object of the present application which is defined in claim 1 and concerns a therapeutic bed the patient support base of which is made of transparent material, preferable Perspex or an other material with identical or similar characteristics and equipped with a video camera, a computer and a monitor which enables the patient to participate actively in the rehabilitation treatment, permitting him/her to see his/her own vertebral disease in the dynamic phase and to see its modifications under the suggestions and guidance of the therapist, thereby optimizing the results obtained.

### Detailed description

Further features and advantages of the invention shall be better understood from the description of a preferred but not exclusive embodiment of the present invention, illustrated by way of indicative but non-limiting example in the accompanying drawings in which:

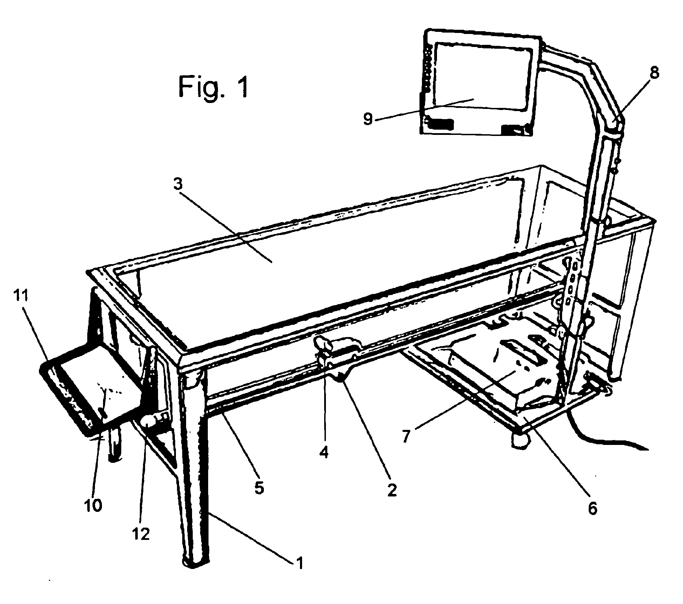

Fig.1 is a complete view of the bed with all of its elements which are object of the claims of the present patent application;

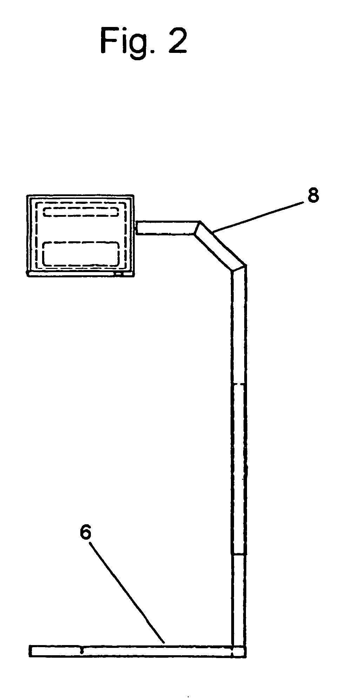

Fig. 2 is a view of the computer trolley with directable arm on which the monitor is to be fitted;

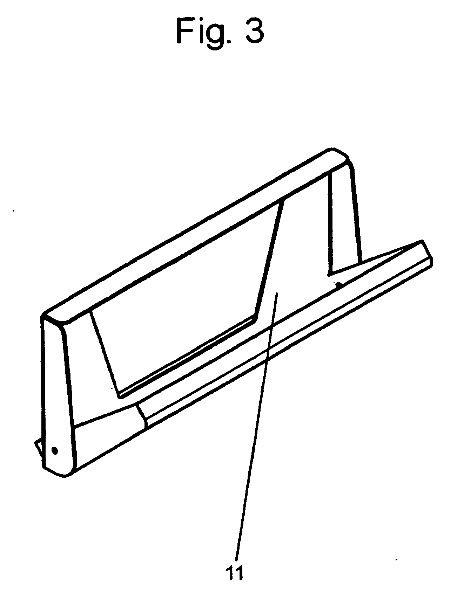

Fig. 3 is a view of the tiltable keyboard holder (11) which is part of the bed;

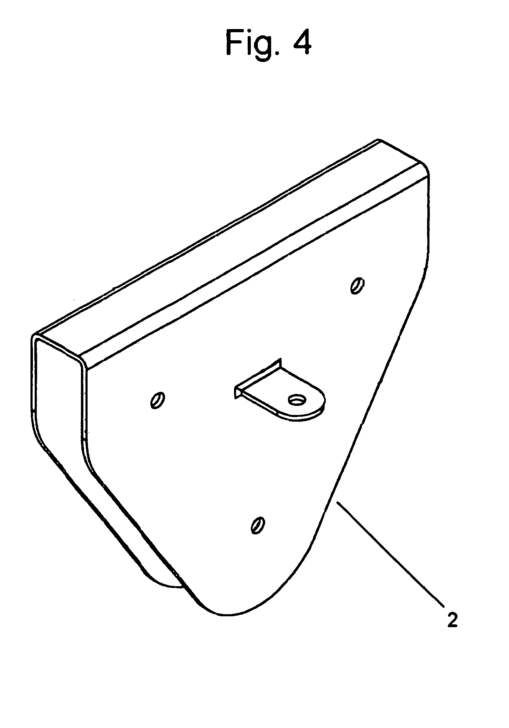

Fig. 4 is a view of the mobile trolley on which the video camera is installed.

The invention that is object of the present application concerns a bed (1) for therapeutic sessions the patient support base (3) of which is transparent, being made of Perspex or other material having identical or similar characteristics. Underneath the patient support base (3) a video camera (4) is installed on a trolley (2) which runs along a rail (5) that is the same length as the transparent surface (3) of the bed (1). The bed is also equipped with a mobile trolley (6) which is used as a computer (7) holder over which there is a directable arm (8) on which a monitor (9) is fitted, positioned in front of the patient who is lying in a supine position and on which the back of the latter is displayed, dynamically filmed by the video camera (4), thereby allowing him/her to better comprehend the exercises suggested by the therapist and executed under the guidance of the same so that he/she might self-assess and self-correct the position of his/her own vertebral column.

On the side opposite the monitor and behind the patient is the therapist's operating position which contains a keyboard (10) connected to the computer (7) housed in a tiltable keyboard holder (11) which is part of the bed (1) and a knob (12) by means of which the therapist, depending on the requirements of the patient's rehabilitation phase as shown on the monitor (9), moves the trolley (2), on which is mounted the video camera (4) positioned underneath the transparent material (3) on which the patient's back rests, by means of two pulleys joined by a cable that runs inside the rail (5).

The present invention is an instrument that facilitates the work of the therapist who, being able to view the vertebral disease his/her own patient in the dynamic phase on the monitor (9) is able to operate therapeutically not, as before, on the basis of the sensations expressed by the patient but on the basis of an objective examination of the part under examination.

The materials and the dimensions of the above-described invention, illustrated in the accompanying drawings and later claimed, may be varied according to requirements. Moreover, all the details may be replaced by other technically equivalent ones without for this reason straying from the protective scope of the present invention.

## Claims

1. Transparent bed (1) for the self-correction of the spinal column through a video camera and a computer system concerning the medical sector and more specifically that of the rehabilitation of the spinal column through a therapist, the bed (1) comprising: a patient support base (3) made of transparent material, a video camera (4) installed under the patient support base (3), a trolley (2) on which the video camera (4) is mounted, a computer (7) and a monitor (9) adapted to display an image of the back of the patient as detected by the video camera (4), when the patient is lying in a supine position on the patient support base (3), a therapist's operating post, located on a side of the support base opposite the monitor, said post containing a keyboard (10) connected to the computer (7) and housed in a tiltable keyboard holder (11), which is part of the bed, and a knob (12) by means of which the therapist can move the trolley (2) by means of two pulleys joined by a cable that runs inside a rail (5).

## Patentansprüche

1. Transparentes Bett (1) zur Selbstkorrektur der Wirbelsäule über eine Videokamera und ein Computersystem für den medizinischen Bereich, insbesondere die Rehabilitation der Wirbelsäule durch einen Therapeuten. Das Bett (1) ist durch folgende Merkmale gekennzeichnet: eine Liegefläche für den Patienten (3) aus transparentem Material, eine unter der Liegefläche für den Patienten (3), auf einer Laufkatze (2) angebrachte Videokamera (4), einen für die Anzeige des durch die Kamera (4) aufgenommenen Bildes des Rücken des Patienten in Rückenlage auf der Liegefläche für den Patienten (3) eingerichteten Computer (7) und Bildschirm (9), ein Bedienpult für den Therapeuten auf einer Seite der Liegefläche für den Patienten, gegenüber des Bildschirms. Jenes Bedienpult enthält eine an den Computer (7) angeschlossene, in einer kippbaren Halterung (11) untergebrachte Tastatur (10), die Teil des Bettes ist, sowie einen Drehknopf (12), mit dem der Therapeut die Laufkatze (2) über zwei durch ein innerhalb einer Schiene (5) laufendes Kabel verbundene Riemenscheiben bewegen kann.

## Revendications

1. Lit transparent (1) pour l'auto-correction de la colonne spinale par un système de caméra vidéo et d'ordinateur concernant le secteur médical et plus spécifiquement celui de la rééducation de la colonne spinale par un thérapeute, le lit (1) comprenant: une base de support de patient (3) composée d'un matériau transparent, une caméra vidéo (4) installée sous la base de support de patient (3), un chariot (2) sur lequel la caméra vidéo (4) est installée, un ordinateur (7) et un moniteur (9) adapté pour afficher une image du dos du patient telle qu'elle est détectée par la caméra vidéo (4), lorsque le patient est allongé sur le dos sur la base de support de patient (3), un poste de travail pour le thérapeute, situé sur un côté de la base de support face au moniteur, ledit poste contenant un clavier (10) raccordé à l'ordinateur (7) et renfermé dans un support pour clavier inclinable (11), qui fait partie du lit, et une molette (12) au moyen de laquelle le thérapeute peut déplacer le chariot (2) par le biais de deux poulies réunies par un câble qui se déplace à l'intérieur d'un rail (5).
